Europäisches Patentamt

European Patent Office

Office européen des brevets

(11) Publication number: **0 131 142**
**A2**

# (12) EUROPEAN PATENT APPLICATION

(21) Application number: **84106321.7**

(22) Date of filing: **02.06.84**

(51) Int. Cl.⁴: **C 12 N 1/00**
**C 07 K 3/02**

(30) Priority: **22.06.83 SE 8303578**

(43) Date of publication of application:
**16.01.85 Bulletin 85/3**

(84) Designated Contracting States:
**BE CH DE FR GB IT LI LU NL**

(71) Applicant: **Gambro Lundia AB**
**Box 10101**
**S-220 10 Lund(SE)**

(72) Inventor: **Kronvall, Göran**
**Tornhem, Östra Torn**
**S-223 68 Lund(SE)**

(72) Inventor: **Björck, Lars**
**Skogsvägen 20A**
**S-240 17 Södra Sandby(SE)**

(74) Representative: **Boberg, Nils Gunnar Erik et al,**
**Gambro AB Patent Department Box 10101**
**S-220 10 Lund(SE)**

(54) **Process for recovering a cell wall protein.**

(57) Process for recovering a cell wall protein, particularly a Fc-receptor, from streptococcal bacteria, wherein proteolytic enzymes are used for solubilization of said protein.

Papain, trypsin and pepsin, preferably papain, are examples of useful proteolytic enzymes for said solubilization.

Fc-receptor type III (protein G) having selective IgG-binding capability (i.e. no albumin-binding capability) can be reovered from streptococcal bacteria, if these bacteria prior to said enzymatic solubilization are treated with enzymes. For this pretreatment it is preferred to use proteolytic enzymes, among which pepsin and trypsin are the most preferred examples.

EP 0 131 142 A2

TITLE

PROCESS FOR RECOVERING A CELL WALL PROTEIN

TECHNICAL FIELD

This invention relates to a process for recovering a cell wall protein from streptococcal bacteria, which process comprises enzymatic solubilization possibly followed by isolation of the solubilized protein.

Particularly the invention relates to a process of the above-mentioned kind for recovering a Fc-receptor from such bacteria.

BACKGROUND OF INVENTION

Fc-receptors from bacteria and processes for recovering these are known and described in the literature.

For example, U.S. Patent 3 850 798 describes a process for recovering the Fc-receptor protein A from staphylococcal bacteria through enzymatic solubilization with the use of the enzyme trypsin.

A similar process using fag-induced cell wall-decomposing enzyme has also been suggested for solubilization of Fc-reactive protein from group A streptococci.

The latter process constitutes one example of the principle that by decomposing the long sugar polymers and the cross-linking peptide-bridges in the cell wall a solubilization of all substances which were bonded to this structure will result. Rests of the cell wall may be left on the solubilized molecules. Simultaneously with the solubilization also all soluble intracellular substances from the streptococcal bacteria are released in the obtained liquid, which therefore will be very complex.

The former process, which according to said U.S. patent can be used in connection with staphylococcal bacteria, is a more selective process. However, this

2

process according to our knowledge has neither been used nor described in connection with streptococcal bacteria. On the contrary, it should have been deemed to be non-operating in view of the results which presently are known with respect to the protein nature of Fc-receptors and the sensitivity of these surface structures to proteolytic treatment. As a rule a complete degradation of the proteins to amino acids and peptides is obtained, and all biological activity is lost.

In contrast to such previous experience it has according to the present invention quite surprisingly been shown that said former process can be used also in connection with streptococcal bacteria, and in particular it has been shown that said process is particularly useful for recovering Fc-receptor type III (desginated protein G in the following) from such bacteria.

Fc-receptors such as protein A and protein G have useful characteristics, for example the ability to bind to the Fc-portion of immunoglobulins, and can advantageously be used as well in therapeutic as in analytical connections. One example of such a useful application is extracorporeal treatment of blood in connection with some autoimmune diseases, wherein the Fc-receptor could be used as an agent to remove so-called immune complexes from the blood. In comparison to protein A protein G has however several advantages which make protein G far superior. For example, protein G can bind to all IgG-subclasses, while protein A cannot bind to human IgG III. Furthermore, protein G lacks the ability to bind IgA and IgM, whereby protein G in a sense is a more selective Fc-receptor compared to protein A which binds also to these immunoglobulin classes.

According to our present knowledge exists no simple available process for recovering Fc-receptors from strep-tococcal bacteria, particularly protein G, and the need to find such a process therefore must be great.

DESCRIPTION OF INVENTION

According to the invention it is thus provided a process for recovering a cell wall protein from streptococcal bacteria, which process comprises enzymatic solubilization, possibly followed by isolation of the solubilized protein. The process is characterized in that said solubilization is carried with the use of a proteolytic enzyme.

The proteolytic enzyme according to the invention can be chosen among papain, trypsin and pepsin. Papain is the preferred enzyme.

As already mentioned, the recovered Fc-receptor from streptococcal bacteria can bind to all IgG-classes. Experiments have shown that the Fc-receptor (protein G) also binds to albumin which is a useful protein in blood plasma. This means that a competition between IgG and albumin to the binding surfaces on protein G thus can exist, when protein G is used in connection with an extracorporeal treatment of blood to remove immune complexes, as previously mentioned.

According to the invention, it has been found that a still more selective protein G, i.e. a protein G which binds only to IgG but not to albumin, can be obtained from streptococcal bacteria, if these bacteria prior to the solubilization are exposed to an enzymatic pretreatment.

For this enzymatic pretreatment according to the invention preferably proteolytic enzymes are used, among which trypsin and pepsin presently are the most preferred examples.

The solubilized cell wall protein can be isolated and recovered in the way as described in the European Patent Publication No. 0 046 915. Briefly, in this known method the solubilized protein, possibly after filtration of bigger impurities, is brought into contact with a ligand, which is immobilized on a soluble carrier, having affinity to said protein to form a complex which is then purified from possible smaller impurities and thereafter split to release the protein. The released protein is then separated and recovered through filtration. For further details of this known method reference is made to said European patent publication.

A similar method, which can also be used according to the invention, is described in the Swedish Patent Application No. 8302638-5.

According to the invention the enzymes are preferably used in the form of a suspension, whereby preferred enzyme suspensions contain between 50 and 250, preferably 75 -150 µg, enzyme per ml of a 10% bacteria suspension.

The invention will be further illustrated with reference to the following non-limiting practical examples.

Example 1

**Enzymatic solubilization**

Human group G streptococcal bacteria, G 148, cultured in Todd/Hewitt-broth, were suspended (10% suspension) in 0.01 M tris-HCl, pH 8.0. 100 µl 0.4 M L-cystein and 10 µl papain of varying concentration in the same buffer were added per ml bacterial suspension. The mixture was incubated for 1 h at 37°C. Jodoacetamide was added to a final concentration of 6 mM.

**Isolation**

The so obtained bacterial suspension was centrifuged (2000 g) for 30 min, whereafter the supernatant was ultracentrifugated (50000 g), frozen immediately at -80°C and used as starting material for isolation of the protein through sequential use of ion exchange chromatography on DEAE-cellulose gel, gel filtration on Sephadex G-100 and affinity chromatography on Sepharose 4B-bonded IgG.

## Analysis

The isolated protein migrated as a homogeneous band when analysed on SDS-PAGE, and on agarose gel electrophoreses only one band was seen in the alfa$_1$-region, indicating a high degree of purity.

SDS-PAGE in disks and in rods (labeled protein G) both gave an apparent molecular weight of 30500. Treatment of the protein with 2-merkaptoethanol did not influence the result.

## Example 2

### Enzymatic pretreatment

0.5 mg pepsin per ml 10% bacterial suspension (streptococcal bacteria of the same kind as above) was combined in 0.1 M acetate buffer, pH 4.0, and the mixture was incubated for 30 min at 37°C. Tests according to known methods on the remaining bacterial bodies showed that these completely lacked the ability to bind albumin, while the ability to bind IgG was maintained unaffected.

### Enzymatic solubilization

The so pepsin-treated bacteria were washed in 0.1 M tris-HCl, pH 8.0, and diluted to a 10% suspension in this buffer. Thereafter, the enzymatic solubilization with papain was performed in the same way as described above to recover a protein G having the ability to selectively bind IgG.

## INDUSTRIAL APPLICABILITY

The process according to the invention can be used to recover cell wall proteins, particularly Fc-receptors from streptococcal bacteria, and is particularly useful to recover Fc-receptor type III (protein G) from streptococcal bacteria.

CLAIMS

1. Process for recovering a cell wall protein from streptococcal bacteria, which process comprises enzymatic solubilization, possibly followed by isolation of the solubilized protein, characterized in that said solubilization is carried out with the use of a proteolytic enzyme.

2. Process according to claim 1, characterized in that the proteolytic enzyme is chosen among papain, trypsin and pepsin, preferably papain.

3. Process according to claim 1 or 2, characterized in that the streptococcal bacteria are pretreated with an enzyme prior to said enzymatic solubilization.

4. Process according to claim 3, characterized in that the pretreatment is carried out with the use of proteolytic enzyme.

5. Process according to claim 3 or 4, characterized in that the proteolytic enzyme for sid pretreatment is chosen among trypsin and pepsin.

6. Process according to any of the preceding claims, characterized in that solubilized protein is isolated through affinity chromatography with the use of a ligand having affinity for the protein.

7. Process according to any of the preceding claims, characterized in that the enzymes for the enzymatic solubilization are used in the form of a suspension.

8. Process according to claim 7, characterized in that the enzymes are used in an amount of between 50 and 250 µg, preferably 75 - 150 µg, enzyme per ml 10% cell suspension.